# EUROPÄISCHE PATENTSCHRIFT

(11) **EP 1 305 275 B1**
(45) Veröffentlichungstag und Bekanntmachung des Hinweises auf die Patenterteilung: **17.11.2004**
(21) Anmeldenummer: 01958028.1
(22) Anmeldetag: 27.07.2001
(51) Int. Cl.: C07C 67/04, C07C 69/54, C07C 69/14

(54) **VERFAHREN ZUR HERSTELLUNG VON TERT.-BUTYLESTERN ALIPHATISCHER C1-C4-CARBONSÄUREN**
METHOD FOR PRODUCING TERT-BUTYL ESTERS OF ALIPHATIC C1-C4-CARBOXYLIC ACIDS
PROCEDE DE PRODUCTION DE TERT-BUTYLESTERS D'ACIDES CARBOXYLIQUES C1-C4 ALIPHATIQUES

(30) Priorität: 28.07.2000 DE 10036959
(43) Veröffentlichungstag der Anmeldung: 02.05.2003
(73) Patentinhaber: BASF AKTIENGESELLSCHAFT, 67056 Ludwigshafen (DE)
(72) Erfinder: KROKER, Ruprecht, 67240 Bobenheim-Roxheim (DE); NESTLER, Gerhard, A/1070 Wien (AT); SCHMITT, Werner, 67227 Frankenthal (DE); SCHUMM, Winfried, 45886 Gelsenkirchen (DE)
(74) Vertreter: Pohl, Michael, Dr.
(86) Internationale Anmeldenummer: PCT/EP2001/008710
(87) Internationale Veröffentlichungsnummer: WO 2002/010109

(56) Entgegenhaltungen:
- EP-A- 0 268 999
- DE-B- 1 128 428

## Beschreibung

Die vorliegende Erfindung betrifft ein kontinuierliches Verfahren zur Herstellung des tert.-Butylesters einer aliphatischen C₁-C₄-Carbonsäure durch Umsetzung der Carbonsäure mit Isobuten.

Das erfindungsgemäße Verfahren führt zu tert.-Butylestern von aliphatischen C₁-C₄-Carbonsäuren. Derartige Ester finden vielfältige Anwendung. Die tert.-Butylester von gesättigten aliphatischen Carbonsäuren wie tert.-Butylacetat, finden beispielsweise als Lösungsmittel Anwendung. Tert.-Butylester der (Meth)acrylsäure sind wichtige Ausgangsstoffe zur Herstellung von Polymerisaten, die u. a. als Bestandteil von Anstrichdispersionen, Klebstoffen oder Lackharzen Anwendung finden. Die Herstellung von tert.-Butylestern erfolgt im Allgemeinen durch säurekatalysierte Addition der entsprechenden Carbonsäuren an Isobuten (Houben-Weyl, Methoden der Organischen Chemie, Bd. 8, 1952, S. 534; US 3,031,495 und US 3,082,246). Als Katalysatoren verwendet man dabei im Reaktionsgemisch lösliche Säuren, z. B. Mineralsäuren oder Alkyl- bzw. Arylsulfonsäuren (DE-A-12 49 857, US 3,087,962, US 3,088,969) oder unlösliche Katalysatoren wie saure Austauscherharze (US 3,037,052, US 3,031,495, DE-A-31 05 399, EP-A-268 999).

Die Umsetzung der Carbonsäuren mit Isobuten erfolgt in der Regel in herkömmlichen Reaktionsbehältern oder in Säulen (DE-A-11 28 428), wobei die Durchmischung des Reaktionsgemisches durch Rühren oder den eingeleiteten Isobuten-Strom erfolgt. Die Wärmeabfuhr erfolgt in üblicher Weise.

Das erhaltene Reaktionsgemisch wird zunächst vom Katalysator befreit. Bei Verwendung eines im Reaktionsgemisch löslichen Katalysators erfolgt dies im Allgemeinen durch Waschen mit Wasser und/oder Neutralisation mit einer wässrigen Alkalilösung (DE-A-11 28 428) oder destillativ (DE-A-12 49 857). Das vom Katalysator befreite Reaktionsgemisch wird anschließend destillativ aufgearbeitet.

In der Regel treten folgende Schwierigkeiten bei der Herstellung von tert.-Butylestern auf:
- Oligomerisierung des Isobutens
- im Falle der Verwendung von α,β-ethylenisch ungesättigten Carbonsäuren Polymerisation der Carbonsäuren oder der Ester bei thermischer Belastung
- Rückspaltung der tert.-Butylester unter Wärmeeinwirkung und/oder in Anwesenheit von Spuren an starken Säuren
- unzureichende Abfuhr der bei der stark exothermen Veresterungsreaktion auftretenden Reaktionswärme, was Rückspaltung des Esters, Oligomerisierung des Isobutens und bei Verwendung einer α,β-ethylenisch ungesättigten Carbonsäure Polymerisation der Carbonsäure und des erhaltenen Esters zur Folge haben kann. Letzteres führt zu Verschmutzung der Apparaturen, Verstopfen von Leitungen und Pumpen und Belegung von Kolonnenböden und Wärmetauscherflächen, was ein unwirtschaftliches und umweltbelastendes Reinigen der Anlagen zur Folge hat.

Im Stand der Technik wurden zahlreiche Versuche unternommen, die auftretenden Schwierigkeiten zu verringern oder zu vermeiden. So kann die Oligomerisierungsneigung von Isobuten durch Erniedrigung der Reaktionstemperatur (US 3,172,905), durch die Anwesenheit von Wasser (US 3,088,969) und durch teilweise Neutralisation des Katalysators (DE-A 31 05 399) verringert werden. Diese Maßnahmen haben jedoch den Nachteil, dass die Reaktionsgeschwindigkeit reduziert wird.

Die Bildung der Isobutenoligomeren kann auch durch die Verwendung von gasförmigem Isobuten verringert werden (DE-A-11 35 897). Nachteilig ist dabei jedoch die notwendige Verdampfung des flüssigen Isoolefins und das Handhaben großer Gasmengen.

Zur Vermeidung bzw. Reduzierung der Polymerisation von α,β-ethylenisch ungesättigten Verbindungen, insbesondere von (Meth)acrylverbindungen werden häufig Polymerisationsinhibitoren, wie Phenothiazin, Hydrochinon oder tert.-Butylbrenzcatechin oder Gemische davon, ggf. unter gleichzeitiger Zugabe von Luft, zugesetzt.
Die Polymerisatbildung lässt sich dadurch aber nicht vollständig verhindern.

Trotz aller Bemühungen wurde bisher aber kein Verfahren bekannt, das die o. g. Schwierigkeiten zumindest so stark verringert, dass daraus ein vorteilhaftes industrielles Verfahren resultiert.

Der vorliegenden Erfindung liegt daher die Aufgabe zugrunde, ein technisch einfaches, wirtschaftliches und umweltschonendes Verfahren zur Herstellung von tert.-Butylestern von aliphatischen Carbonsäuren zur Verfügung zu stellen.

Überraschenderweise wurde nun gefunden, dass diese Aufgabe gelöst wird, wenn man die Umsetzung der Carbonsäuren mit Isobuten in einem Reaktor mit mehreren Abschnitten durchführt und die Reaktionstemperatur in jedem Abschnitt so kontrolliert, dass sie im Reaktor im Bereich von 10 bis 40°C liegt und vorteilhaft im ersten Abschnitt am höchsten ist.

Die vorliegende Erfindung betrifft daher ein Verfahren zur Herstellung des tert.-Butylesters einer aliphatischen C₁-C₄-Carbonsäure durch Umsetzung der Carbonsäure mit Isobuten in flüssiger Phase in Gegenwart eines sauren Katalysators und Gewinnung des Esters aus dem erhaltenen Reaktionsgemisch, wobei man die Umsetzung in einem in mehrere Abschnitte unterteilten Reaktor durchführt und die Carbonsäure, das Isobuten und den Katalysator in die erste Zone des Reaktors einspeist, wobei die Reaktionstemperatur im Reaktor so kontrolliert wird, dass sie im Bereich von 10 bis 40°C liegt und im ersten Abschnitt am höchsten ist.

Bei den aliphatischen C₁-C₄-Carbonsäuren handelt es sich insbesondere um Ameisensäure, Essigsäure, Propionsäure, Buttersäure, Isobuttersäure, Acrylsäure oder Methacrylsäure, wobei Acrylsäure oder Methacrylsäure besonders bevorzugt sind.

Das Isobuten wird vorzugsweise in flüssiger Form in den ersten Abschnitt des Reaktors eingespeist. Das Mengenverhältnis von Carbonsäure zu Isobuten kann in einem weiteren Bereich variieren. Vorzugsweise jedoch verwendet man die Carbonsäure im Überschuss (auf molarer Basis). Vorzugsweise liegt das Molverhältnis von Carbonsäure: Isobuten im Bereich von 1,1:1 bis 1,5:1.

Das Verfahren erfolgt im Allgemeinen in Abwesenheit eines Lösungsmittels. Als Katalysatoren verwendet man daher solche, die im Reaktionsgemisch zumindest teilweise löslich sind. Insbesondere die anorganischen Katalysatoren sind zu Beginn der Umsetzung nur teilweise im Reaktionsgemisch löslich. Derartige Katalysatoren sind also vor allem im ersten und zweiten Abschnitt teilweise im Reaktionsgemisch fein dispergiert. Im Laufe der Umsetzung wird der Katalysator besser löslich (in erster Linie auf Grund der Bildung eines Teilesters des Katalysators, z. B. des Schwefelsäurehalbesters). Zumindest im letzten Abschnitt liegt er daher im allgemeinen im Reaktionsgemisch gelöst vor. Brauchbare Katalysatoren sind starke anorganische oder organische Säuren, wie Mineralsäuren beispielsweise Schwefelsäure, Phosphorsäure und Polyphosphorsäure, vorzugsweise Schwefelsäure oder Sulfonsäuren, wie p-Toluol-, Benzol-, Dodecylbenzol-, und Methansulfonsäure.

Die Katalysatormenge beträgt im Allgemeinen etwa 0,1 bis 10 Gew.-%, vorzugsweise 0,5 bis 5 Gewichtsprozent, bezogen auf die Einsatzstoffe Carbonsäure und Isobuten.

Die Umsetzung wird im Allgemeinen auch in Gegenwart eines Inhibitors durchgeführt, der die Polymerisation der ungesättigten Carbonsäure oder des Esters hemmt. Besonders geeignete Inhibitoren sind Hydrochinon, Hydrochinonmonomethylether, p-Benzochinon, p-Nitrosophenol, Phenothiazin, 4-Hydroxy-2,2,6,6-tetramethyl-1-oxyl-piperidin und Methylenblau. Die Inhibitoren kommen im Allgemeinen in Mengen von etwa 200 bis 2000 ppm, bezogen auf das Gewicht der Einsatzstoffe Carbonsäure und Isoolefin, zur Anwendung.

Das erfindungsgemäße Verfahren wird in einem Reaktor durchgeführt, bei dem es sich insbesondere um einen zylindrischen Reaktor handelt. Der Reaktor ist in mehrere, vorzugsweise 3, 4 oder 5, voneinander getrennte Abschnitte unterteilt. Die Abschnitte werden durch Trennwände, die senkrecht zur Längsachse des Reaktors verlaufen, voneinander getrennt. Diese weisen jeweils mindestens eine Öffnung auf, um den Durchtritt des Reaktionsgemisches von einem Reaktorabschnitt zum nächsten zu ermöglichen. Die Zahl der Öffnungen pro Trennwand richtet sich nach der Größe des Reaktors. Vorzugsweise weisen die Trennwände eine Öffnung auf, die sich insbesondere in der Mitte der Trennwand befindet. Die Gesamtfläche der Öffnungen pro Trennwand beträgt etwa 1/2000 bis 1/500 der Reaktorquerschnittsfläche.

Das Volumen der Reaktorabschnitte kann gleich oder verschieden sein. Vorzugsweise ist das Volumen des ersten Reaktorabschnitts größer als das der restlichen Abschnitte. Bei einem Reaktor mit vier Abschnitten haben sich folgende Anteile der einzelnen Abschnitte am Gesamtreaktorvolumen als bevorzugt erwiesen:

| | |
|---|---|
| Reaktorabschnitt 1 | 25 bis 50% |
| Reaktorabschnitt 2 | 10 bis 25% |
| Reaktorabschnitt 3 | 10 bis 25% |
| Reaktorabschnitt 4 | 25 bis 50% |

Die Reaktorabschnitte können vorteilhaft mit Einbauten ausgestattet sein, um die Durchmischung des Reaktionsgemisches zu verbessern. Geeignete Einbauten sind dem Fachmann bekannt, beispielsweise kann es sich um statische Mischelemente, wie Gitterroste, Verteilerbleche oder Siebböden, handeln. Besonders bevorzugt ist es, den ersten Reaktorabschnitt mit derartigen Einbauten auszurüsten, die insbesondere in der oberen Hälfte des Reaktorabschnittes vorgesehen werden.

Die Einsatzstoffe Carbonsäure und Isobuten werden in flüssiger Form in den ersten Abschnitt des Reaktors eingespeist, insbesondere im Bereich des Bodens des Reaktors. Die Einspeisung kann direkt, z. B. über ein Tauchrohr erfolgen, es ist jedoch bevorzugt, Mittel vorzusehen, welche eine gleichmäßige Verteilung und Durchmischung der Einsatzstoffe ermöglichen. Derartige Mittel sind dem Fachmann bekannt, beispielsweise handelt es sich um Verteilerplatten, perforierte Platten und Rohre, Düsen etc. Das Isobuten wird vorzugsweise über ein ringförmiges Rohr mit mehreren Austrittsöffnungen eingespeist. Die Carbonsäure wird vorzugsweise über eine Düse eingespeist, welche die Vermischung eines Gases und einer Flüssigkeit und die Durchmischung des Reaktorinhalts bewirkt. Sie ist vorzugsweise im Boden des Reaktors angeordnet. Geeignete Düsen sind dem Fachmann bekannt (Strahldüse, Mischdüse, Zweistoffdüse etc.) und z. B. in Ullmann's Encyclopedia of Industrial Chemistry, Vol. B4, 5. ED., 1992, S. 280 beschrieben. Insbesondere bei Verwendung einer derartigen Düse ist die Strömung in den beiden ersten Reaktorabschnitten turbulent und in den folgenden Reaktorabschnitten im Wesentlichen laminar.

Es hat sich als vorteilhaft erwiesen, die frische Carbonsäure im Gemisch mit dem Rückstand der Katalysatorabtrennung und dem Rückstand der Reindestillation (siehe unten) in den Reaktor einzuspeisen. Außerdem hat es sich als vorteilhaft erwiesen, bei der Destillation des Esters anfallendes Rück-Isobuten in den ersten Reaktorabschnitt einzuspeisen. Bei Verwendung von gasförmigem Isobuten ist es besonders vorteilhaft, das Isobuten über die erwähnte Düse zusammen mit der Carbonsäure einzuspeisen. Die Düse bewirkt ein selbsttätiges Ansaugen des zurückgeführten gasförmigen Isobutens.

Aus dem ersten und/oder zweiten Reaktorabschnitt kann ein Teil des Reaktionsgemisches entnommen und wieder in den betreffenden Abschnitt zurückgeführt werden. Dadurch wird eine bessere Durchmischung des Reaktionsgemisches gewährleistet. Die Rückführung des Teilstroms erfolgt zweckmäßig über die oben erwähnte Mischdüse in den ersten Reaktorabschnitt und/oder über eine weitere Düse im Bereich der in der Trennwand befindlichen Öffnung in den zweiten Reaktorabschnitt. Bei der weiteren Düse kann es sich um eine Düse des oben für die Mischdüse genannten Typs handeln. Bevorzugt wird eine kegelförmige Düse verwendet. Vorzugsweise ist sie so angeordnet, dass sich ihre Austrittsöffnung etwa in Höhe der Trennwand befindet, die den ersten vom zweiten Abschnitt trennt. Gewünschtenfalls kann der jeweils entnommene Teilstrom zur Kontrolle der Temperatur über einen Wärmetauscher geführt werden.

Das erhaltene Reaktionsgemisch wird am oberen Ende des Reaktors entnommen und der weiteren Aufarbeitung zugeführt. Unumgesetztes, gasförmiges Isobuten sammelt sich im oberen Bereich des Reaktors an. Dieses wird in den ersten Reaktorabschnitt zurückgeführt, vorzugsweise über die erwähnte Düse am Boden des ersten Reaktorabschnitts. Vorzugsweise wird der aus dem Reaktor kommende isobutenhaltige Gasstrom durch Kondensation des Isobutens von inerten Gasen, wie Luft und Butan, befreit. Das Isobuten wird dann in flüssiger Form über die Mischdüse oder zusammen mit dem Frisch-Isobuten in den ersten Reaktorabschnitt eingespeist.

Der Katalysator wird im Gemisch mit der Carbonsäure eingespeist, wobei frischer Katalysator oder zurückgewonnener Katalysator oder ein Gemisch davon zur Anwendung kommen kann.

Die Reaktionstemperatur liegt insgesamt im Bereich von etwa 10 bis 40°C. Sie wird vorzugsweise so gesteuert, dass sie im ersten Reaktorabschnitt am höchsten ist. Vorzugsweise liegt die Reaktionstemperatur im ersten Reaktorabschnitt im Bereich von etwa 30 bis 40°C. Im zweiten Abschnitt ist sie niedriger, vorzugsweise um etwa 5 bis 15°C. Die Temperatur in den nach dem zweiten Abschnitt folgenden Abschnitten kann gleich oder verschieden sein. Sie ist im Allgemeinen nicht höher als im zweiten Abschnitt, vorzugsweise ist sie niedriger, insbesondere um etwa 3 bis 10°C. Im vierten Abschnitt ist sie im Allgemeinen so hoch wie im dritten Abschnitt oder um etwa 1 bis 5°C niedriger. Die Temperatur im letzten Reaktorabschnitt liegt vorzugsweise im Bereich von etwa 10 bis 25°C.

Die Temperaturverteilung in einem Reaktor mit 4 Abschnitten ist vorzugsweise wie folgt:

| | | |
|---|---|---|
| 1. | Abschnitt | 33 - 38°C |
| 2. | Abschnitt | 23 - 28°C |
| 3. | Abschnitt | 15 - 22°C |
| 4. | Abschnitt | 15 - 22°C |

Die Temperatur im 3. und 4. Abschnitt kann dabei gleich oder verschieden sein.

Da die Addition von Carbonsäuren an Isobuten stark exotherm ist, ist es zur Einstellung der Reaktionstemperatur zweckmäßig, zumindest in den ersten beiden Reaktorabschnitten die Reaktionswärme abzuführen. Dies erfolgt insbesondere mit Hilfe von Wärmetauschern, die außen- oder innenliegend ausgeführt sein können. Auch eine Kühlung der Reaktorwände ist möglich. Es hat sich als zweckmäßig erwiesen, die Temperaturkontrolle in den beiden ersten Reaktorabschnitten mit Hilfe von außenliegenden Wärmetauschern vorzunehmen, über die ein Teilstrom des in dem jeweiligen Reaktorabschnitt befindlichen Reaktionsgemisches geführt und wieder zurückgeführt wird.

Das erfindungsgemäße Verfahren kann mit Überdruck, Unterdruck oder vorzugsweise drucklos oder unter leichtem Überdruck (100 - 300 mbar) durchgeführt werden.

Das aus dem Reaktor austretende Reaktionsgemisch enthält einen hohen Anteil an dem gewünschten Ester. Daneben enthält es unumgesetzte Einsatzstoffe, Katalysator, Inhibitor, Ester der Katalysatorsäure und weitere geringfügige Nebenprodukte. Das Reaktionsgemisch enthält nur sehr geringe Mengen an Isobuten-Oligomerisierungsprodukt, im Allgemeinen ≤ 2 Gew.-%, bezogen auf das Reaktionsgemisch.

Zur Gewinnung von Reinester wird das Reaktionsgemisch einer weiteren Aufarbeitung unterzogen. Die Aufarbeitung ist in keiner Weise beschränkt und kann nach jedem zu diesem Zweck üblichen Verfahren erfolgen. Man geht dabei zweckmäßiger Weise so vor, dass zunächst der Katalysator abgetrennt wird. Dies kann durch ein oder mehrmaliges Waschen des Reaktionsgemisches mit Wasser und/oder durch Neutralisation des Katalysators mit einer wässrigen Alkalilösung (beispielsweise Natronlauge, Kalilauge, wässrige Natrium- oder Kaliumcarbonatlösung oder -bicarbonatlösung) durchgeführt werden. Alternativ kann die Katalysatorabtrennung durch Destillation des Reaktionsgemisches erfolgen, beispielsweise in einer Destillationseinheit aus Verdampfer, Kolonne und Kondensator. Man erhält auf diese Weise ein Kopfprodukt, das im Wesentlichen den Zielester, geringe Mengen an Carbonsäure und leichtsiedende Bestandteile (tert.-Butanol und Diisoolefin) umfaßt, und ein Sumpfprodukt, das den Katalysator, die Hauptmenge der unumgesetzten Carbonsäure und die hochsiedenden Bestandteile, z. B. polymere (Meth)acrylverbindungen, umfasst. Das Sumpfprodukt wird im Allgemeinen zumindest teilweise in den Reaktor zurückgeführt.

Im Anschluß an die Katalysatorabtrennung erfolgt die Abtrennung der Leichtsieder in einer üblichen Destillationseinheit aus Verdampfer, Kolonne und Kondensator. Der von den Leichtsiedern befreite Rohester bleibt als Sumpfprodukt zurück und wird dann in üblicher Weise einer Reindestillation unterworfen. Der Rückstand der Reindestillation, hauptsächlich Carbonsäure und etwas Ester, wird in den ersten Reaktionsabschnitt zurückgeführt.

Das erfindungsgemäße Verfahren besitzt folgende Vorteile:
- Einfache und wirksame Kontrolle der Reaktionstemperatur, so dass die Nebenproduktbildung, insbesondere die Bildung von Oligomerisierungsprodukten des Isobutens äußerst gering ist;
- das Isobuten wird flüssig eingesetzt, so dass keine engergieaufwendige Verdampfung des Isobutens erforderlich ist;
- der Reaktor ist technisch einfach aufgebaut, so dass die Investitionskosten gering sind;
- der Reaktor ist aufgrund seiner einfachen Bauweise und des Fehlens von bewegten Teilen wartungsarm, so dass die Betriebskosten gering sind;
- zurückgeführtes Isobuten (Abgas aus den Aufarbeitungsstufen) kann gasförmig wiederverwendet werden, so dass keine Verflüssigung erforderlich ist;
- alle carbonsäurehaltigen Rückstände können gemeinsam zurückgeführt werden;
- die Verwendung von Düsen, die mit den Einsatzstoffen bzw. den Rückströmen angetrieben werden, bewirken gute Durchmischung des Reaktionsgemisches;
- das Verfahren ermöglicht die problemlose Rückführung des Katalysators

Das erfindungsgemäße Verfahren läßt sich nicht nur mit Isobuten durchführen. Es ist vorteilhaft bei Verwendung eines Isoolefins der Formel 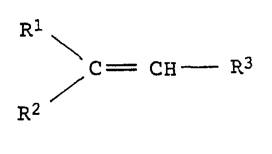 worin
R¹ und R² unabhängig voneinander für Methyl oder Ethyl stehen und R³ für H, Methyl oder Ethyl steht. Man erhält dabei einen tert.-C₄-C₈-Alkylester einer aliphatischen C₁-C₄-Carbonsäure.

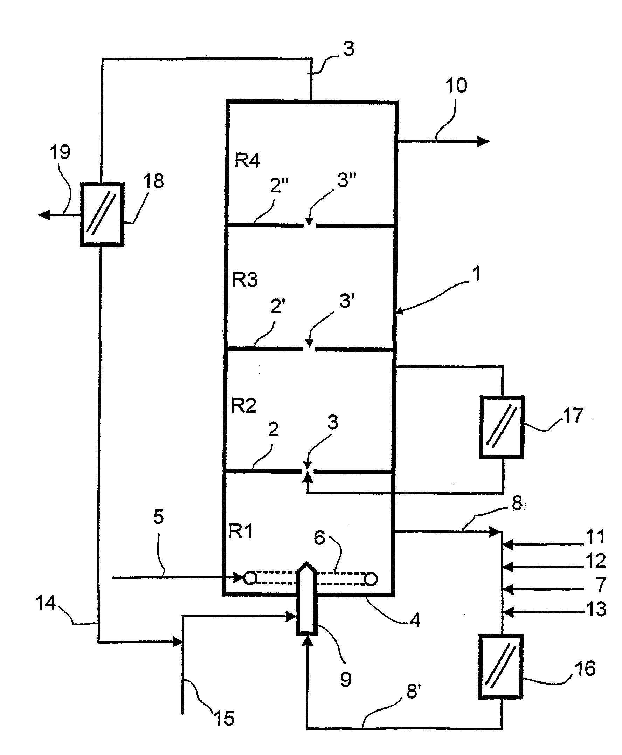

Das erfindungsgemäße Verfahren wird nachfolgend rein beispielhaft zur Herstellung von tert.-Butylacrylat unter Bezug auf die Figur und ohne darauf beschränkt zu sein erläutert.

Die Figur zeigt eine schematische Darstellung eines axialen Längsschnittes durch den erfindungsgemäßen Reaktor.

Der Reaktor 1 mit einem Gesamtvolumen von etwa 10 m³ (Durchmesser etwa 1,3 m) ist in vier Abschnitte R1, R2, R3, R4 unterteilt, die durch Trennwände 2, 2', 2'' voneinander getrennt sind. In der Mitte jeder Trennwand befindet sich eine runde Öffnung 3, 3', 3'' (Durchmesser bis zu etwa 5 cm), die den Durchtritt des Reaktionsgemisches in die nächste Kammer erlaubt. Volumen der Reaktorabschnitte am Gesamtvolumen: R1 = etwa 40%, R2 = etwa 15%,
R3 = etwa 15% und R4 = etwa 30%. Der erste Reaktorabschnitt R1 ist in der oberen Hälfte mit einem (nicht gezeigten) Gitterrost ausgestattet.

Frisches Isobuten wird über Leitung 5 im Bereich des Bodens 4 des Reaktors 1 bzw. des Reaktorabschnitts R1 in flüssiger Form eingespeist (500 l/h). Die Verteilung des frischen Isobutens erfolgt durch ein im unteren Bereich des Reaktorabschnitts R1 symmetrisch angeordnetes ringförmiges Rohr 6 mit mehreren (nicht gezeigten) Austrittsöffnungen. Über die Leitung 8' und die zentrisch angeordnete Strahldüse 9 wird ein Gemisch in den unteren Bereich des Reaktorabschnittes R1 eingespeist, das aus frischer Acrylsäure 7 (430 l/h), dem Sumpf 11 der Katalysatorabtrennung (1150 l/h, Acrylsäuregehalt ca. 40 Gew.-%), dem Sumpf 12 der Reindestillation (320 l/h, Acrylsäuregehalt ca. 70 Gew.-%), dem frischen Katalysator 13 (3,5 l/h, Schwefelsäure 98%ig), dem Rück-Isobuten 14 aus dem Reaktorabgas, dem Austrag 8 aus dem Reaktorabschnitt R1 (ca 75 m³/h) und dem gasförmigen Rück-Isobuten 15 (92 m³/h), das bei den Destillationen im Rahmen der Aufarbeitung des erhaltenen Reaktionsgemisches anfällt, besteht. Die Strahldüse 9 saugt dabei das gasförmige Rück-Isobuten 15 und das flüssige Rückisobuten 14 an.

Durch das Rohr 6 und die Düse 9 wird eine intensive Vermischung der Einsatzstoffe bewirkt. Um die Vermischung noch weiter zu verbessern wird im oberen Bereich des Reaktorabschnitts R1 ein Teil des Reaktionsgemisches ausgetragen und im Kreislauf wieder über die Düse 9 in den Reaktorabschnitt R1 zurückgeführt. In den ausgetragenen Teilstrom 8 werden die erwähnten Ströme 7 und 11 bis 13 eingemischt. Der Gesamtstrom wird über den Wärmetauscher 16 geleitet, um die Reaktionswärme abzuführen und die Temperatur im ersten Reaktorabschnitt auf etwa 35°C einzustellen.

Das Reaktionsgemisch aus dem Reaktor R1 gelangt durch die Öffnung 3 (Durchmesser etwa 3 cm) in den Reaktorabschnitt R2. Im oberen Bereich des Reaktorabschnitts R2 wird ein Teil des Reaktionsgemisches (4 m^{3/}h) ausgeschleust und zur Abfuhr der Reaktionswärme über den Wärmetauscher 17 geleitet und über eine (nicht gezeigte) kegelförmige Düse wieder in den Reaktorabschnitt R2 zurückgeführt. Die Düse ist so angeordnet, dass sich ihre Austrittsöffnung in der Öffnung 3 etwa in der Ebene der Trennwand 2 befindet. Die Reaktionstemperatur in R2 wird auf diese Weise auf etwa 25°C eingestellt. Durch die von der erwähnten Düse bewirkte zusätzliche Strömung wird der Fluß des Reaktionsgemisches von R1 nach R2 unterstützt und der Inhalt von R2 intensiv durchmischt. Der Übertritt des Reaktionsgemisches von R2 nach R3 und von R3 nach R4 erfolgt über die Öffnungen 3' bzw. 3" (Durchmesser jeweils 2 cm). Die Wärmeabfuhr in R3 und R4 erfolgt jeweils über (nicht gezeigte) innenliegende Lamellenkühler, wobei die Temperatur jeweils auf 20°C eingestellt wird. Das Reaktionsgemisch (2,4 m³/h) wird aus dem Reaktorabschnitt R4 über eine Flüssigkeitsstandregelung über Leitung 10 ausgetragen und der weiteren Aufarbeitung zugeführt.

Die sich oberhalb der Flüssigphase im Reaktorabschnitt R4 bildende Gasphase wird am Kopf des Reaktors ausgeschleust und über einen mit Sole betriebenen Kühler 18 geleitet, um das im Abgas enthaltene Isobuten zu kondensieren und von den inerten Gasen (2,5 m³/h, hauptsächlich Butane und Luft) zu trennen. Die inerten Gase werden über die Leitung 19 entsorgt und das flüssige Isobuten wird über die Düse 9 wieder in den Reaktorabschnitt R1 zurückgeführt.

Der Reaktoraustrag hat im Wesentlichen folgende Zusammensetzung:

| | |
|---|---|
| Tert.-Butylacrylat | 64,0 Gew.-% |
| Tert.-Butylacetat | 0,2 Gew.-% |
| Tert.-Butanol | 1,6 Gew.-% |
| Acrylsäure | 24,1 Gew.-% |
| Isobuten | 2,5 Gew.-% |
| Diisobuten | 1,2 Gew.-% |
| Schwefelsäure | 1,0 Gew.-% |
| Tert.-Butylschwefelsäure | 5,3 Gew.-% |
| Phenothiazin | 0,1 Gew.-% |

Zur Aufarbeitung wird der Reaktoraustrag mit Hilfe eines Dünnschichtverdampfers (80°C, 60 mbar) in ein Destillat, das hauptsächlich tert. Butylacrylat, tert. Butylacetat, Butanol und Acrylsäure enthält, und ein Sumpfprodukt, das im Wesentlichen Acrylsäure, Katalysator und Phenothiazin enthält und das in den Reaktor zurückgeführt wird, aufgetrennt. Das Destillat wird mit 1000 ppm Phenothiazin und 500 ppm p-Nitrosophenol versetzt, in einer weiteren üblichen Destillationskolonne (40 Böden, Sumpftemperatur 78°C, Kopftemperatur 38°C bei 115 mbar) in ein Kopfprodukt (Leichtsieder), hauptsächlich tert. Butylacetat, Diisobuten und tert. Butanol, und ein Sumpfprodukt, hauptsächlich tert. Butylacrylat und Acrylsäure aufgetrennt. Das Destillat wird teilweise ausgeschleust (3 Vol. %) und teilweise, mit 200 ppm Phenothiazin und 100 ppm p-Nitrosophenol versetzt, als Rücklauf in den Kolonnenkopf zurückgeführt. In einer weiteren Destillationskolonne bekannter Bauart (40 Böden, Sumpftemperatur 92°C, Kopftemperatur 49°C bei 75 mbar) wird das tert. Butylacrylat als Kopfprodukt gewonnen, wobei das Sumpfprodukt, das ca. 70 Gew.-% Acrylsäure enthält, in den Reaktor zurückgeführt wird. Die bei den Destillationsschritten anfallenden isobutenhaltigen Abgase werden vereinigt und dem Reaktor wieder zugeführt. Das kondensierte tert. Butylacrylat wird mit 15 ppm Hydrochinonmonomethylether stabilisiert, teilweise (ca. 50 %) als Rücklauf auf den obersten Boden der Kolonne aufgebracht und teilweise ausgeschleust. Das auf diese Weise gewonnene tert. Butylacrylat hat eine Reinheit von 99,9 %.

## Patentansprüche

1. Verfahren zur kontinuierlichen Herstellung des tert.-Butylesters einer aliphatischen C₁-C₄-Carbonsäure durch Umsetzung der Carbonsäure mit Isobuten in flüssiger Phase in Gegenwart eines sauren Katalysators, wobei man die Umsetzung in einem in mehrere Abschnitte unterteilten Reaktor durchführt, die Carbonsäure, das Isoolefin und den Katalysator in den ersten Abschnitt des Reaktors einspeist, das erhaltene Reaktionsgemisch aus dem letzten Abschnitt des Reaktors entnimmt und den Ester daraus gewinnt, wobei man die Reaktionstemperatur im Reaktor so kontrolliert, dass sie im Bereich von 10 bis 40°C liegt und im ersten Abschnitt des Reaktors am höchsten ist.

2. Verfahren nach Anspruch 1, wobei man einen Reaktor mit 3 bis 5 Abschnitten verwendet.

3. Verfahren nach Anspruch 1 oder 2, wobei die Reaktionstemperatur im ersten Abschnitt im Bereich von 30 bis 40°C liegt.

4. Verfahren nach einem der vorhergehenden Ansprüche, wobei die Reaktionstemperatur im zweiten Abschnitt um 5 bis 15°C niedriger ist als im ersten Abschnitt.

5. Verfahren nach Anspruch 4, wobei die Reaktionstemperatur im dritten Abschnitt um 3 bis 10°C niedriger ist als im zweiten Abschnitt.

6. Verfahren nach einem der vorhergehenden Ansprüche, wobei die Reaktionstemperatur im ersten Abschnitt des Reaktors im Bereich von 30 bis 40°C und im letzten Abschnitt des Reaktors im Bereich von 10 bis 25°C liegt.

7. Verfahren nach einem der vorhergehenden Ansprüche, wobei man einen Teil des im ersten Abschnitt enthaltenen Reaktionsgemisches austrägt und zusammen mit Carbonsäure wieder dem ersten Abschnitt zuführt.

8. Verfahren nach einem der vorhergehenden Ansprüche, wobei das in den ersten Abschnitt eingespeiste Isobuten frisches und rückgeführtes Isobuten umfasst, wobei das frische Isobuten in flüssiger Form und das rückgeführte Isobuten in flüssiger Form und/oder gasförmig eingespeist wird.

9. Verfahren nach Anspruch 8, wobei das rückgeführte Isobuten im Gemisch mit der Carbonsäure eingespeist wird.

10. Verfahren nach einem der vorhergehenden Ansprüche, wobei man als Carbonsäure (Meth)acrylsäure oder Essigsäure verwendet.

11. Reaktor, der in mehrere Abschnitte (R1, R2, R3, R4) unterteilt ist, die durch Trennwände (2, 2', 2'') mit jeweils mindestens einer Öffnung (3, 3', 3'') voneinander getrennt sind und der eine in den ersten Abschnitt (R1) reichende Düse (9), Mittel zum Zuführen und Abführen der beteiligten Stoffe und Mittel zur Kontrolle der Temperatur in den Abschnitten (R1, R2, R3, R4) aufweist.

12. Reaktor nach Anspruch 11, wobei die Trennwände (2, 2', 2'') jeweils eine Öffnung (3, 3', 3'') aufweisen, die sich im Wesentlichen in der Mitte der Trennwand befindet.

13. Reaktor nach Anspruch 11 oder 12, wobei man als Düse 9 eine Strahldüse, Mischdüse oder Zweistoffdüse verwendet.

14. Reaktor nach einem der Ansprüche 11 bis 13, wobei im ersten Abschnitt (R1) im Bereich des Bodens ein ringförmiges Rohr (6) mit mehreren Austrittsöffnungen und einer Leitung (5) zur Zufuhr eines Einsatzstoffes vorgesehen ist.

15. Reaktor nach einem der Ansprüche 11 bis 14, wobei das Volumen des ersten Abschnittes (R1) größer ist als das der übrigen Abschnitte und 25 bis 50% des Gesamtreaktorvolumens ausmacht.

16. Reaktor nach einem der Ansprüche 11 bis 15, wobei der erste und/oder zweite Abschnitt (R1, R2) mit statischen Mischelementen ausgestattet ist.

17. Reaktor nach einem der Ansprüche 11 bis 16, wobei eine Düse zur Vermischung des Inhalts des zweiten Abschnitts (R2) vorgesehen ist, die so angeordnet ist, dass sich ihre Austrittsöffnung in der Öffnung (3) etwa in der Ebene der Trennwand (2) befindet.

## Claims

1. A process for the continuous preparation of the tert-butyl ester of an aliphatic C₁-C₄-carboxylic acid by reacting the carboxylic acid with isobutene in the liquid phase in the presence of an acidic catalyst, wherein the reaction is carried out in a reactor divided into a plurality of sections, the carboxylic acid, the isoolefin and the catalyst are fed into the first section of the reactor, the reaction mixture obtained is removed from the last section of the ' reactor and the ester is obtained therefrom, the reaction temperature in the reactor being controlled so that it is from 10 to 40°C and is highest in the first section of the reactor.

2. A process as claimed in claim 1, wherein a reactor having from 3 to 5 sections is used.

3. A process as claimed in claim 1 or 2, wherein the reaction temperature in the first section is in the range of from 30 to 40°C.

4. A process as claimed in any of the preceding claims, wherein the reaction temperature in the second section is from 5 to 15°C lower than that in the first section.

5. A process as claimed in claim 4, wherein the reaction temperature in the third section is from 3 to 10°C lower than that in the second section.

6. A process as claimed in any of the preceding claims, wherein the reaction temperature in the first section of the reactor is in the range of from 30 to 40°C and that in the last section of the reactor is in the range of from 10 to 25°C.

7. A process as claimed in any of the preceding claims, wherein a part of the reaction mixture contained in the first section is discharged and is recycled together with carboxylic acid to the first section.

8. A process as claimed in any of the preceding claims, wherein the isobutene fed into the first section comprises fresh and recycled isobutene, the fresh isobutene being fed in in liquid form and the recycled isobutene being fed in in liquid form and/or gaseous form.

9. A process as claimed in claim 8, wherein the recycled isobutene is fed in as a mixture with the carboxylic acid.

10. A process as claimed in any of the preceding claims, wherein the carboxylic acid used is (meth)acrylic acid or acetic acid.

11. A reactor which is divided into a plurality of sections (R1, R2, R3, R4), which are separated from one another by dividing walls (2, 2', 2'') each having at least one orifice (3, 3', 3''), and which has a nozzle (9) extending into the first section (R1), means for feeding in and removing the substances involved and means for controlling the temperature in the sections (R1, R2, R3, R4).

12. A reactor as claimed in claim 11, wherein the dividing walls (2, 2', 2'') each have an orifice (3, 3', 3'') which is present substantially in the center of the dividing wall.

13. A reactor as claimed in claim 11 or 12, wherein the nozzle 9 used is a jet nozzle, mixing nozzle or binary nozzle.

14. A reactor as claimed in any of claims 11 to 13, wherein an annular tube (6) having a plurality of outlet orifices and a line (5) for feeding in a starting material is provided in the region of the bottom in the first section (R1).

15. A reactor as claimed in any of claims 11 to 14, wherein the volume of the first section (R1) is greater than that of the remaining sections and accounts for from 25 to 50% of the total reactor volume.

16. A reactor as claimed in any of claims 11 to 15, wherein the first and/or second section (R1, R2) is equipped with static mixing elements.

17. A reactor as claimed in any of claims 11 to 16, wherein a nozzle for mixing the content of the second section (R2) is provided and is arranged in such a way that its outlet orifice is present in the orifice (3), roughly in the plane of the dividing wall (2).

## Revendications

1. Procédé de production en continu de l'ester tert-butylique d'un acide carboxylique aliphatique en C₁ à C₄ au moyen de la réaction de l'acide carboxylique avec de l'isobutène en phase liquide, en présence d'un catalyseur acide, dans lequel on réalise la réaction dans un réacteur divisé en plusieurs sections, on introduit l'acide carboxylique, l'iso-oléfine et le catalyseur dans la première section du réacteur, on évacue le mélange réactionnel obtenu à partir de la dernière section du réacteur et on en extrait l'ester, en régulant la température de réaction dans le réacteur de manière à ce qu'elle soit située dans la gamme de 10°C à 40°C et qu'elle soit la plus élevée dans la première section du réacteur.

2. Procédé selon la revendication 1, dans lequel on utilise un réacteur comprenant 3 à 5 sections.

3. Procédé selon la revendication 1 ou 2, dans lequel la température de réaction dans la première section va de 30°C à 40°C.

4. Procédé selon l'une quelconque des revendications précédentes, dans lequel la température de réaction dans la deuxième section est inférieure de 5°C à 15°C par rapport à celle de la première section.

5. Procédé selon la revendication 4, dans lequel la température de réaction dans la troisième section est inférieure de 3°C à 10°C par rapport à celle de la deuxième section.

6. Procédé selon l'une quelconque des revendications précédentes, dans lequel la température de réaction dans la première section du réacteur va de 30°C à 40°C et dans la dernière section du réacteur de 10°C à 25°C.

7. Procédé selon l'une quelconque des revendications précédentes, dans lequel on soutire une partie du mélange réactionnel contenu dans la première section et on le recycle conjointement avec l'acide carboxylique dans la première section.

8. Procédé selon l'une quelconque des revendications précédentes, dans lequel l'isobutène introduit dans la première section comprend de l'isobutène frais et de l'isobutène recyclé, l'isobutène frais étant introduit sous forme liquide et l'isobutène recyclé étant introduit sous forme liquide et/ou gazeuse.

9. Procédé selon la revendication 8, dans lequel l'isobutène recyclé est introduit sous forme de mélange avec l'acide carboxylique.

10. Procédé selon l'une quelconque des revendications précédentes, dans lequel on utilise, en tant qu'acide carboxylique, l'acide (méth)acrylique ou l'acide acétique.

11. Réacteur, qui est divisé en plusieurs sections (R1, R2, R3, R4), qui sont séparées les unes des autres par des cloisons de séparation (2, 2', 2") présentant chacune au moins une ouverture (3, 3', 3"), et qui présente une buse (9) arrivant dans la première section (R1), des moyens pour introduire et évacuer les matières mises en oeuvre et des moyens pour réguler la température dans les sections (R1, R2, R3, R4).

12. Réacteur selon la revendication 11, dans lequel les cloisons de séparation (2, 2', 2") présentent chacune une ouverture (3, 3', 3") qui se trouve essentiellement au milieu de la cloison de séparation.

13. Réacteur selon la revendication 11 ou 12, dans lequel on utilise, en tant que buse 9, une buse d'éjection, une buse mélangeuse ou une buse de substance binaire.

14. Réacteur selon l'une quelconque des revendications 11 à 13, dans lequel est prévu, dans la première section (R1), dans la zone du fond, un tube annulaire (6) comprenant plusieurs orifices de sortie et une conduite (5) pour l'introduction d'un produit de départ.

15. Réacteur selon l'une quelconque des revendications 11 à 14, dans lequel le volume de la première section (R1) est plus important que celui des sections restantes et représente 25% à 50% du volume total du réacteur.

16. Réacteur selon l'une quelconque des revendications 11 à 15, dans lequel la première et/ou la deuxième section (R1, R2) est équipée d'éléments mélangeurs statiques.

17. Réacteur selon l'une quelconque des revendications 11 à 16, dans lequel est prévue une buse pour mélanger le contenu de la deuxième section (R2), qui est disposée de manière à ce que son orifice de sortie dans l'ouverture (3) se trouve approximativement sur le même plan que la cloison de séparation (2).
